# EUROPEAN PATENT APPLICATION

(11) **EP 3 932 353 A1**
(43) Date of publication of application: **05.01.2022**
(21) Application number: 21165851.3
(22) Date of filing: 30.03.2021
(51) Int. Cl.: A61B 18/14

(54) **ESTIMATING PROGRESS OF IRREVERSIBLE ELECTROPORATION ABLATION BASED ON AMPLITUDE OF MEASURED BIPOLAR SIGNALS**

(30) Priority: 29.06.2020 US 202016914624
(71) Applicant: Biosense Webster (Israel) Ltd, Yokneam, 2066717 (IL)
(72) Inventor: ALTMANN, Andres Claudio, 2066717 Yokneam (IL); GOVARI, Assaf, 2066717 Yokneam (IL); ZILBERMAN, Israel, 2066717 Yokneam (IL)
(74) Representative: Carpmaels & Ransford LLP

(57) **Abstract**

A method includes, coupling, to a target tissue, at least a pair of electrodes of an ablation catheter; at a first stage of an ablation procedure, a first bipolar signal having a first amplitude, is received from the pair of electrodes; at a second stage of the ablation procedure, a second bipolar signal having a second amplitude, is received from the pair of electrodes; based on the first and second amplitudes, at least a parameter indicative of a progress of the ablation procedure is estimated.

## Description

### FIELD OF THE INVENTION

The present invention relates generally to tissue ablation procedures, and particularly to methods and systems for estimating parameters in irreversible electroporation ablation procedures.

### BACKGROUND OF THE INVENTION

Various techniques for tracking progress of tissue ablation using a given electrode are known in the art.

For example, U.S. Patent No. 10,342,606 describes high-frequency ablation of tissue in the body using a cooled high-frequency electrode connected to a high frequency generator including a computer graphic control system and an automatic controller for control the signal output from the generator, and adapted to display on a real time graphic display a measured parameter related to the ablation process and visually monitor the variation of the parameter of the signal output that is controlled by the controller during the ablation process.

### SUMMARY OF THE INVENTION

An exemplary embodiment of the present invention that is described herein provides a method including coupling, to a target tissue, at least a pair of electrodes of an ablation catheter; at a first stage of an ablation procedure, a first bipolar signal having a first amplitude, is received from the pair of electrodes; at a second stage of the ablation procedure, a second bipolar signal having a second amplitude, is received from the pair of electrodes; based on the first and second amplitudes, at least a parameter indicative of a progress of the ablation procedure is estimated.

In some exemplary embodiments, estimating the parameter includes displaying the first and second amplitudes on a same graph. In other exemplary embodiments, displaying the first and second amplitudes includes displaying, on the same graph, a first bar indicative of the first amplitude and a second bar indicative of the second amplitude. In yet other exemplary embodiments, the method includes receiving from an additional pair of electrodes of the ablation catheter, an additional first bipolar signal having an additional first amplitude and an additional second bipolar signal having an additional second amplitude, and the method further includes, displaying on the same graph an additional first bar indicative of the additional first amplitude, and an additional second bar indicative of the additional second amplitude.

In an exemplary embodiment, the pair of electrodes is coupled to the target tissue at a first site, and the additional pair of electrodes is coupled to the target tissue at a second site, different from the first site, and the method includes, based on the same graph, applying, to the target tissue: (i) a first irreversible electroporation (IRE) pulse at the first site, and (ii) a second IRE pulse at the second site. In another exemplary embodiment, applying the first and second IRE pulses includes applying the first IRE pulse that differs from the second IRE pulse. In yet another exemplary embodiment, displaying the first and second amplitudes includes displaying the first and second bars overlaying one another.

In some exemplary embodiments, receiving at the first stage includes receiving the first bipolar signal before applying an irreversible electroporation (IRE) pulse to the target tissue, and receiving at the second stage includes receiving the second bipolar signal after applying the IRE pulse to the target tissue. In other exemplary embodiments, the method includes, based on the estimated parameter, checking whether to apply to the target tissue, an additional IRE pulse, and in case the additional IRE pulse is required, the method includes, at a third stage of the ablation procedure: (i) based on the estimated parameter, applying to the target tissue the additional IRE pulse, (ii) receiving, from the pair of electrodes, a third bipolar signal having a third amplitude, and (iii) estimating at least the parameter based on the first and third amplitudes. In yet other exemplary embodiments, estimating the parameter includes displaying at least one of: (i) a first numerical difference between the first and second amplitudes, and (ii) a second numerical difference between the second amplitude and a target threshold.

There is additionally provided, in accordance with an exemplary embodiment of the present invention, a system including a processor and a display. The processor is configured to receive, from at least a pair of electrodes of an ablation catheter that are coupled to a target tissue: (a) at a first stage of an ablation procedure, a first bipolar signal having a first amplitude, and (b) at a second stage of the ablation procedure, a second bipolar signal having a second amplitude, and based on the first and second amplitudes, the processor is configured to estimate at least a parameter indicative of a progress of the ablation procedure. The display is configured to display the parameter.

### BRIEF DESCRIPTION OF THE DRAWINGS

The present invention will be more fully understood from the following detailed description of the exemplary embodiments thereof, taken together with the drawings in which:
Fig. 1 is a schematic, pictorial illustration of a catheter-based position-tracking and irreversible electroporation (IRE) ablation system, in accordance with an exemplary embodiment of the present invention;
Fig. 2 is a schematic pictorial illustration of a graph of amplitudes displayed for estimating and monitoring one or more IRE ablation parameters, in accordance with an exemplary embodiment of the present invention; and
Fig. 3 is a flow chart that schematically illustrates a method for estimating one or more ablation parameters based on amplitude of bipolar signals acquired during an IRE ablation procedure, in accordance with an exemplary embodiment of the present invention.

### DETAILED DESCRIPTION OF EMBODIMENTS

### OVERVIEW

Irreversible electroporation (IRE) may be used, for example, for treating arrhythmia by ablating tissue cells using high-voltage pulses applied to the tissue. Cellular destruction occurs when the transmembrane potential exceeds a threshold, leading to cell death and formation of a lesion. In IRE-based ablation procedures, high-voltage bipolar electrical pulses are applied, for example, to one or more pairs of electrodes in contact with tissue to be ablated, so as to form a lesion between the electrodes, and thereby to treat arrhythmia in a patient's heart.

Sometimes, more than one set of bipolar electrical pulses, also referred to herein as IRE pulses, are required for forming the lesion, and thereby, to conclude the ablation procedure. Moreover, the lesion intended to be ablated may be non-uniform, so that a different amount of IRE pulses may be required to be applied to the tissue by different pairs of electrodes.

In principle, after applying the IRE pulses, it is possible to measure impedance between the electrodes of each pair, so as to receive an indication of whether or not to conclude the ablation procedure. However, such impedance measurements may not provide sufficiently accurate indication of the progress of the ablation procedure.

Exemplary embodiments of the present invention that are described hereinbelow provide improved techniques for estimating one or more parameters related to the progress of the ablation procedure.

In some exemplary embodiments, a physician inserts an IRE ablation catheter into an ablation site having tissue intended to be ablated in a patient's heart. The ablation catheter comprises one or more pairs of electrodes, which are in contact with heart tissue at the ablation site.

In some exemplary embodiments, an IRE ablation system comprises an IRE pulse generator (IPG), a switching box, which is configured to switch the power applied by the IPG to one or more selected pairs of electrodes, and a processor.

In some exemplary embodiments, at a first stage of the ablation procedure, e.g., before applying a first set of one or more IRE pulses, the physician may use the pair of electrodes for measuring a first bipolar signal measured at a given site of the tissue at the ablation site. In the context of the present disclosure and in the claims, the term "bipolar signal" refers to any suitable signal, such as but not limited to an impedance. The bipolar signal is measured between two electrodes of the catheter, or electrodes of any other probe at the same site, such as the electrodes of the aforementioned pair. In the context of the present disclosure and in the claims, the term "IRE pulse" and "set of IRE pulses" are used interchangeably and refer to a set of one or more IRE pulses.

In some exemplary embodiments, at a second stage of the ablation procedure, e.g., after applying the first IRE pulse, the physician may use the pair of electrodes for measuring a second bipolar signal measured at the given site.

In some exemplary embodiments, the IRE ablation system comprises a processor, which is configured to receive, from each pair of electrodes, the first and second bipolar signals. The processor is configured to identify, in the first and second bipolar signals received from each pair, first and second amplitudes, respectively.

In some exemplary embodiments, the processor is configured to display, for each pair of electrodes, a suitable graph, such as a bar graph, showing a visual comparison between the first and second amplitudes.

In some exemplary embodiments, based on the displayed graph of amplitudes, the physician can estimate for each pair of electrodes, one or more parameters related to the progress and efficacy of the ablation procedure. For example, based on the displayed graph of amplitudes, the physician may estimate that the first IRE pulse is insufficient for forming the required lesion at the given site. In this example, the physician may use the IPG and the switching box for applying to the tissue, a second IRE pulse solely at the given site.

The disclosed techniques provide the physician, e.g., during the ablation procedure, with a visual indication of the progress of the ablation at each ablation site. Moreover, the disclosed techniques help optimizing the amount of IRE pulses applied to the tissue, and therefore, improves the safety of the process for the patient and reduces the cycle time of the ablation procedure.

### SYSTEM DESCRIPTION

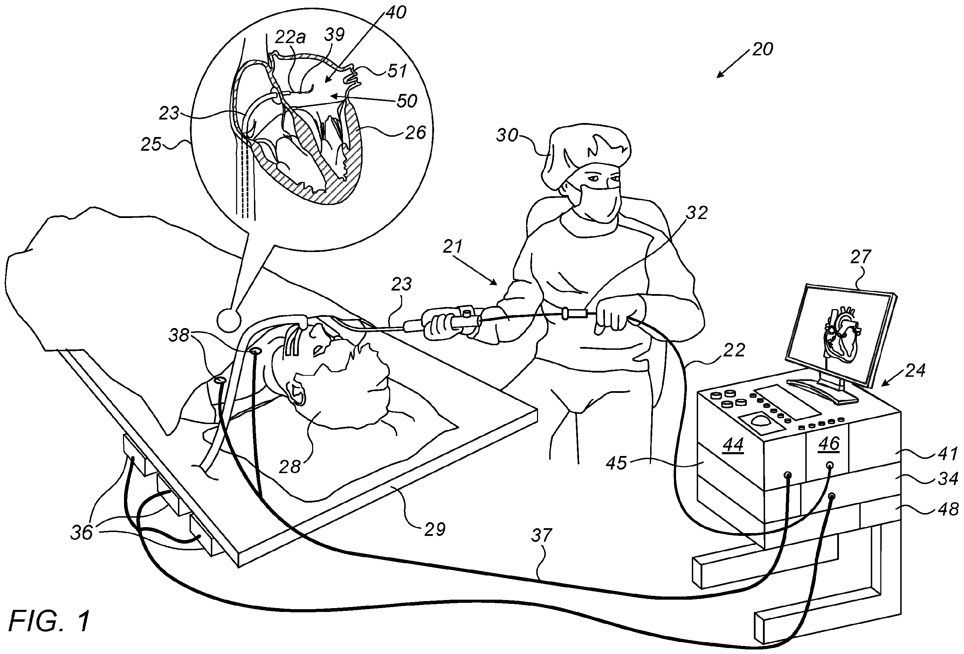

Fig. 1 is a schematic, pictorial illustration of a catheter 21 based position-tracking and irreversible electroporation (IRE) ablation system 20, in accordance with an exemplary embodiment of the present invention.

In some exemplary embodiments, system 20 comprises a deflectable tip section 40 that is fitted at a distal end 22a of a shaft 22 of catheter 21 with deflectable tip section 40 comprising multiple electrodes 50 as illustrated in inset 25.

In the exemplary embodiment described herein, electrodes 50 are configured to sense intra-cardiac (IC) electrocardiogram (ECG) signals, and may additionally be used for IRE ablation of tissue of left atrium of a heart 26, such as IRE ablation of an ostium 51 of a pulmonary vein in heart 26. Note that the techniques disclosed herein are applicable, *mutatis mutandis,* to other sections (e.g., atrium or ventricle) of heart 26, and to other organs of a patient 28.

In some exemplary embodiments, the proximal end of catheter 21 is connected to a control console 24 (also referred to herein as a console 24) comprising an ablative power source, in the present example an IRE pulse generator (IPG) 45, which is configured to deliver peak power in the range of tens of kW. Console 24 comprises a switching box 46, which is configured to switch the power applied by IPG 45 to one or more selected pairs of electrodes 50. A sequenced IRE ablation protocol may be stored in a memory 48 of console 24.

In some exemplary embodiments, a physician 30 inserts distal end 22a of shaft 22 through a sheath 23 into heart 26 of patient 28 lying on a table 29. Physician 30 navigates distal end 22a of shaft 22 to a target location in the heart 26 by manipulating shaft 22 using a manipulator 32 near the proximal end of catheter 21 and/or deflection from sheath 23. During the insertion of distal end 22a, deflectable tip section 40 is maintained in a straightened configuration by sheath 23. By containing tip section 40 in a straightened configuration, sheath 23 also serves to minimize vascular trauma when physician 30 moves catheter 21, through the vasculature of patient 28, to the target location, such as an ablation site in heart 26.

Once distal end 22a of shaft 22 has reached the ablation site, physician 30 retracts sheath 23 and deflects tip section 40, and further manipulates shaft 22 to place electrodes 50 disposed over tip section 40 in contact with the ostium 51 at the ablation site. In the present example, the ablation site comprises a pulmonary vein, but in other exemplary embodiments, physician 30 may select any other suitable ablation site.

In some exemplary embodiments, electrodes 50 are connected by wires running through shaft 22 to a processor 41, which is configured to control switching box 46 of interface circuits 44 in console 24.

As further shown in inset 25, distal end 22a comprises a position sensor 39 of a position tracking system, which is coupled to distal end 22a, e.g., at tip section 40. In the present example, position sensor 39 comprises a magnetic position sensor, but in other exemplary embodiments, any other suitable type of position sensor (e.g., other than magnetic based) may be used. During navigation of distal end 22a in heart 26, processor 41 of console 24 receives signals from magnetic position sensor 39 in response to magnetic fields from external field generators 36, for example, for the purpose of measuring the position of tip section 40 in heart 26 and, optionally, displaying the tracked position overlaid on the image of heart 26, on a display 27 of console 24. Magnetic field generators 36 are placed at known positions external to patient 28, e.g., below table 29. Console 24 also comprises a driver circuit 34, configured to drive magnetic field generators 36.

The method of position sensing using external magnetic fields is implemented in various medical applications, for example, in the CARTO^{™} system, produced by Biosense Webster Inc. (Irvine, Calif.) and is described in detail in U.S. Patent Nos. 5,391,199, 6,690,963, 6,484,118, 6,239,724, 6,618,612 and 6,332,089, in PCT Patent Publication WO 96/05768, and in U.S. Patent Application Publication Nos. 2002/0065455 A1, 2003/0120150 A1 and 2004/0068178 A1, whose disclosures are all incorporated herein by reference.

Typically, processor 41 of console 24 comprises a general-purpose processor of a general-purpose computer, with suitable front end and interface circuits 44 for receiving signals from catheter 21, as well as for applying ablation energy via catheter 21 in a left atrium of heart 26 and for controlling the other components of system 20. Processor 41 typically comprises a software in memory 48 of system 20, which is programmed to carry out the functions described herein. The software may be downloaded to the computer in electronic form, over a network, for example, or it may, alternatively or additionally, be provided and/or stored on non-transitory tangible media, such as magnetic, optical, or electronic memory.

### ESTIMATING EFFICACY AND PROGRESS OF IRREVERSIBLE ELECTROPORATION ABLATION

Irreversible electroporation (IRE), also referred to as Pulsed Field Ablation (PFA), may be used as a minimally invasive therapeutic modality for forming a lesion (e.g., killing tissue cells) at the ablation site by applying high-voltage pulses to the tissue. In the present example, IRE pulses may be used for killing myocardium tissue cells in order to treat cardiac arrhythmia in heart 26. Cellular destruction occurs when the transmembrane potential exceeds a threshold, leading to cell death and thus the development of a tissue lesion. Therefore, of particular interest is the use of high-voltage bipolar electrical pulses, e.g., using a pair of electrodes 50 in contact with tissue at the ablation site, to generate high electric fields (e.g., above a certain threshold) to kill tissue cells located between the electrodes.

In the context of this disclosure, "bipolar" voltage pulse means a voltage pulse applied between two electrodes 50 of catheter 21, as opposed, for example, to unipolar pulses that are applied, e.g., during a radio-frequency ablation, by a catheter electrode relative to some common ground electrode not located on the catheter.

To implement IRE ablation over a relatively large tissue region of heart 26, such as a circumference of an ostium of a pulmonary vein (PV) or any other suitable organ, it is necessary to use multiple pairs of electrodes 50 of catheter 21 having multi electrodes 50 in the deflectable tip section 40. To make the generated electric field as spatially uniform as possible over a large tissue region it is best to have pairs of electrodes 50 selected with overlapping fields, or at least fields adjacent to each other. However, there is a Joule heating component that occurs with the IRE generated fields, and this heating may damage the electrodes when multiple pairs of electrodes 50 are continuously used for delivering a sequence of IRE pulses.

In an exemplary embodiment, system 20 comprises surface electrodes 38, shown in the example of Fig. 1, as attached by wires running through a cable 37 to the chest and shoulder of patient 28. In some exemplary embodiments, surface electrodes 38 are configured to sense body-surface (BS) ECG signals in response to beats of heart 26. Acquisition of BS ECG signals may be carried out using conductive pads attached to the body surface or any other suitable technique. As shown in Fig. 1, surface electrodes 38 are attached to the chest and shoulder of patient 28, however, additional surface electrodes 38 may be attached to other organs of patient 28, such as limbs.

In some exemplary embodiments, electrodes 50 are configured to sense intra-cardiac (IC) ECG signals, and (e.g., at the same time) surface electrodes 38 are sensing the BS ECG signals. In other exemplary embodiments, sensing the IC ECG signals may be sufficient for performing the IRE ablation, so that surface electrodes 38 may be applied for other use cases.

In some exemplary embodiments, physician 30 may couple at least a pair of electrodes 50 to a target tissue at the ablation site in heart 26. The target tissue is intended to be ablated by applying one or more IRE pulses via electrodes 50. Note that the IRE pulses may be applied to the target tissue multiple times, e.g., during different stages of the IRE ablation procedure.

In some exemplary embodiments, before applying the first IRE pulse to the target tissue, referred to herein as a first stage of the IRE ablation procedure, physician 30 may use a pair of electrodes 50 for acquiring a bipolar signal, referred to herein as a first bipolar signal. At subsequent stages, e.g., second and third stages, of the IRE ablation procedure, after applying the first IRE pulse, physician 30 may use the pair of electrodes 50 for acquiring one or more subsequent bipolar signals, e.g., referred to herein as second and third bipolar signals, for monitoring various parameters related to the efficacy and/or progress of the IRE ablation procedure. In the present example, the term "second bipolar signal" refers to a bipolar signal acquired by a pair of electrodes after applying to the target tissue a first IRE pulse, and the term "third bipolar signal" refers to a bipolar signal acquired after applying to the target tissue a second, subsequent, IRE pulse. In other exemplary embodiments, the first, second and third stages may refer to any other stages of the IRE ablation procedure, and therefore, the corresponding first, second and third bipolar signals may be acquired at any other suitable time, e.g., before, during, or after applying the IRE pulses.

In some exemplary embodiments, processor 41 is configured to receive the aforementioned first and second (and optionally third) bipolar signals from electrodes 50, and to hold for each received bipolar signal, the amplitude of the respective bipolar signal. For example, during the IRE ablation procedure, processor 41 may hold (i) a first amplitude of the first bipolar signal (e.g., acquired by electrodes 50 before applying to target tissue the first IRE pulse), (ii) a second amplitude of the second bipolar signal (e.g., acquired by electrodes 50 after applying to target tissue the first IRE pulse), and optionally, (iii) a third amplitude of the third bipolar signal, which may be acquired by electrodes 50 after optionally applying to target tissue the second IRE pulse.

In some exemplary embodiments, based on at least the first and second amplitudes, processor 41 is configured to estimate one or more parameters, such as but not limited to, efficacy and progress of the IRE ablation.

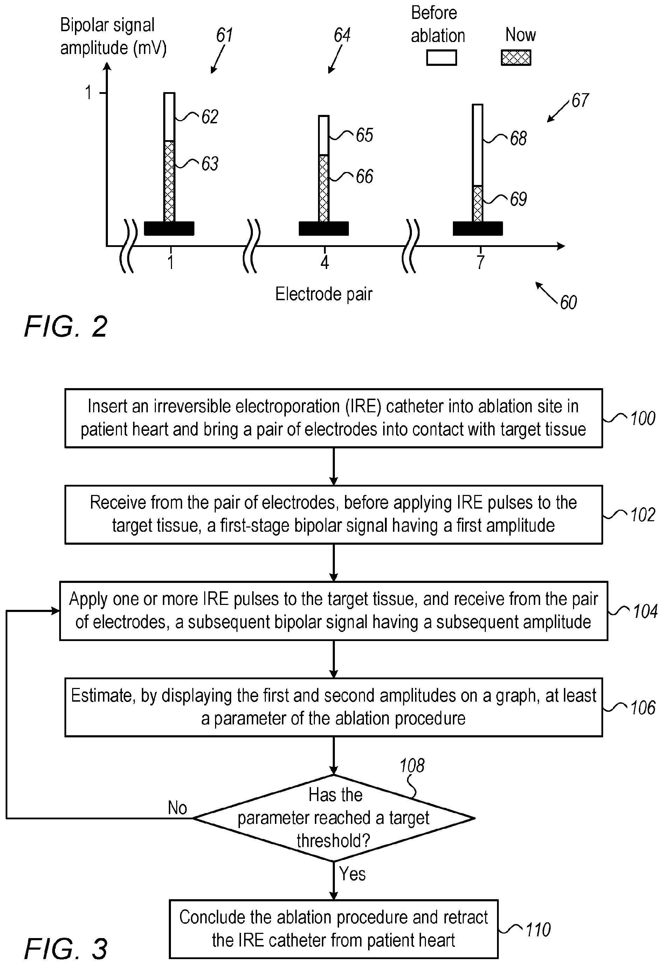

In some exemplary embodiments, processor 41 is configured to display, e.g., on display 27, a graph having two or more of the aforementioned amplitudes. Such graph is described in detail in Fig. 2 below.

This particular configuration of system 20 is shown by way of example, in order to illustrate certain problems that are addressed by exemplary embodiments of the present invention and to demonstrate the application of these exemplary embodiments in enhancing the performance of such an IRE ablation system. Exemplary embodiments of the present invention, however, are by no means limited to this specific sort of example system, and the principles described herein may similarly be applied to other sorts of ablation systems.

### DISPLAYING AMPLITUDE OF MULTIPLE BIPOLAR SIGNALS FOR MONITORING PARAMETERS OF IRREVERSIBLE ELECTROPORATION ABLATION

Fig. 2 is a schematic pictorial illustration of a graph 60 of amplitudes displayed for estimating and monitoring one or more IRE ablation parameters, in accordance with an exemplary embodiment of the present invention. As described in Fig. 1 above, graph 60 may be displayed on display 27 or on any other suitable display of system 20.

In some exemplary embodiments, graph 60 displays three bars 61, 64 and 67, indicative of amplitudes (measured in mv) of bipolar signals received from three respective pairs 1, 4 and 7 of electrodes 50, which physician 30 had coupled to (e.g., brought into contact with) the target tissue of heart 26, for example, during the first and second stages of the IRE ablation procedure.

In the example of Fig. 2, bar 61 comprises bars 62 and 63 overlaying one another, which are indicative of the amplitude of bipolar signals measured between electrodes 50 of pair 1. Bar 62 is indicative of the amplitude of the bipolar signal acquired before applying IRE ablation pulses to the target tissue, also referred to herein as the first bipolar signal. Similarly, bar 63 is indicative of the amplitude of the bipolar signal acquired after applying the latest ablation pulses to the target tissue, also referred to herein as the second bipolar signal. Similarly, bar 64 comprises bars 65 and 66 overlaying one another, which are indicative of the amplitude of bipolar signals measured, respectively, at the aforementioned first and second stages of the IRE ablation procedure, between electrodes 50 of electrodes pair 4. In addition, bar 67 comprises bars 68 and 69 overlaying one another, which are indicative of the amplitude of bipolar signals measured, respectively, at the aforementioned first and second stages of the IRE ablation procedure, between electrodes 50 of electrodes pair 7.

In some exemplary embodiments, based on bars 61, 64 and 67 of graph 60, processor 41 is configured assist physician 30 to estimate one or more parameters related to the progress of the IRE ablation carried out at the ablation site of heart 26. For example, based on graph 60, physician 30 can estimate the efficacy of the IRE ablation, and whether or not he/she needs to use IPG 45 (and switching box 46) for applying additional IRE pulses, also referred to herein as a second IRE pulse, to one or more specific pairs of electrodes 50, so as to improve the outcome IRE ablation procedure. In other exemplary embodiments, processor 41 is configured to control IPG 45 (and switching box 46) for applying additional IRE pulses to one or more specific pairs of electrodes 50. Note that bars 62, 65 and 68 are indicative of the bipolar signal amplitude measured before applying the IRE pulses to the target tissue, and bars 63, 66 and 69 are indicative of the bipolar signal amplitude measured after applying (e.g., a first set of) the IRE pulses to the target tissue.

In some exemplary embodiments, by displaying the difference between amplitudes measured on the same pair of electrodes, physician 30 can estimate the progress of the IRE ablation at the respective ablated area. In the example of Fig. 2, the difference between bars 68 and 69 of bar 67 is larger than the difference between bars 65 and 66 of bar 64. Note that the amplitudes illustrated by bars 64 and 67 are measured on respective electrodes pairs 4 and 7, and bars 64 and 67 are produced by processor 41. By looking at bars 64 and 67, physician 30 can see that, at the area measured by electrodes pair 7 (compared to the area measured by electrodes pair 4), the efficacy of the ablation is larger, and the ablation is progressing faster.

In alternative exemplary embodiments, instead of displaying graph 60, processor 41 is configured to display percentage of reduction in signal amplitude. For example, processor 41 may display the amplitude of bipolar signals measured between electrodes 50 before ablating the target tissue as 100%, and after applying one or more IRE pulses, processor 41 may display the remaining percentage or the amount of reduced percentage. In the example of Fig. 2, in electrodes pair 1 processor 41 may display, instead of the difference between bars 62 and 63, a numerical value such as 35% of reduced percentage, and in electrodes pair 7 processor 41 may display 70%, which corresponds to the difference (in percentage) between bars 68 and 69. In the context of the present disclosure and in the claims, the term reduced percentage may refer to any sort of showing a numerical difference between the measured amplitude before and after applying the one or more IRE pulses.

In other exemplary embodiments, processor 41 is configured to display a threshold for the amplitude level, which is indicative of having sufficient IRE pulses applied to a respective electrodes pair. In the example of Fig. 2, the threshold may be larger than the amplitude displayed on bar 69, and smaller than the amplitude displayed on bars 63 and 66. In other words, there is no need to apply additional IRE pulses to electrodes pair 7, but in order to form the lesion, it is required to apply additional one or more IRE pulses by electrodes pairs 1 and 4. Moreover, based on bars 63 and 66, physician 30 or processor 41 may control IPG 45 to apply different sets of additional one or more IRE pulses to electrodes pairs 1 and 4, so as to form a uniform lesion along the target tissue at the ablation site. For example, a first additional IRE pulse having a given energy and given duration, may be applied to the target tissue by electrodes pair 4, and a second additional IRE pulse having, e.g., a larger energy and/or longer duration, may be applied to the target tissue by electrodes pair 1, or just repeating the same ablation on the subset of the electrodes.

In other exemplary embodiments, processor 41 is configured to set a threshold for each electrodes pair (or a common threshold for all pairs of electrodes), and to display, after applying one or more IRE pulses, (i) the measured amplitude level relative to the threshold (e.g., using a graphical display), or a numerical value (also referred to herein as a numerical difference) showing the arithmetic difference between the threshold and the measured amplitude level. For example, the amplitude before applying the IRE pulses may be 1 mv or 2 mv, however, in case, after applying a first set of the one or more IRE pulses, the measured amplitude is lower than 0.1 mv, there is no need to apply additional IRE pulses to the respective electrodes pair. In other words, it is important to display how far the measured amplitude from the threshold is.

This particular configuration of graph 60 is shown by way of example, in order to illustrate certain problems that are addressed by exemplary embodiments of the present invention and to demonstrate the application of these exemplary embodiments in enhancing the performance of an ablation system. Exemplary embodiments of the present invention, however, are by no means limited to this specific sort of example graph, and the principles described herein may similarly be applied to other sorts of graphs for improving the estimation of ablation parameters during and after an IRE or any other sort of ablation procedure. In other exemplary embodiments, instead of or in addition to graph 60, the measured amplitudes may be displayed using adjacent (rather than overlaying one another) bars, or using any other suitable type of graph, such as but not limited to scattering, so as to estimate one or more parameters indicative of the progress of the ablation procedure. Moreover, physician 30 may select to display one or more types of graphs from a list displayed on display 27. In some exemplary embodiments, processor 41 may hold additional bipolar signals measured at the same location over time, and in response to a command from physician 30, processor 41 is configured to display a comparison between multiple (e.g., more than two) measured amplitudes, so that physician 30 can estimate the progress of the IRE ablation.

In some exemplary embodiments, at a third stage of the ablation procedure, which is carried out after applying the additional IRE pulse to the target tissue, processor 41 is configured to receive, from the respective pair of electrodes, a measurement of a third bipolar signal having a third amplitude. Based on the first amplitude (that was acquired by the pair of electrodes before applying the first IRE pulse) and the third amplitude acquired at the third stage, processor 41 is configured to estimate the one or more parameters indicative of the progress of the ablation procedure.

Fig. 3 is a flow chart that schematically illustrates a method for estimating, based on amplitude of bipolar signals acquired during the IRE ablation procedure, one or more parameters indicative of the progress of the ablation procedure, in accordance with an exemplary embodiment of the present invention.

The method begins at a catheter insertion step 100, with physician 30 inserting IRE catheter 21 into an ablation site in patient heart 26 and bringing one or more pairs of electrodes 50 in contact with target tissue of heart 26. As described in Fig. 1 above, when one or more pairs of electrodes 50 are coupled to the target tissue in heart 26, catheter 21 is configured to carry out the IRE ablation by applying the one or more IRE pulses, which are generated by IPG 45, to the target tissue.

The description below refers to a single pair of electrodes 50, but is also applicable for multiple pairs of electrodes 50, as shown, for example, in Fig. 2 above.

At a first ablation step 102, processor 41 receives from the pair of electrodes 50, at a first stage of the ablation procedure, e.g., before applying the IRE pulses to the target tissue, a first (e.g., pre-ablation) bipolar signal having a first amplitude. At a second ablation step 104, physician 30 uses catheter 21 for applying a first IRE pulse to the target tissue. After applying the first IRE pulse, processor 41 receives from the pair of electrodes 50, a subsequent (e.g., a second) bipolar signal having a subsequent (e.g., second) amplitude. Note that the second bipolar signal is measured between the pair of electrodes 50 at the ablation site, after applying the first IRE pulse to the target tissue.

At an estimation step 106, processor 41 estimates, or assists physician 30 to estimate, e.g., by displaying the first and second amplitudes on graph 60, at least a parameter indicative of the progress of the ablation procedure, such as the efficacy of the IRE ablation. At a decision step 108, processor 41 and or physician 30 checks whether the parameter has reached a target threshold. The term "target threshold" may refer to a threshold number, or to a ratio or difference between the first and second amplitudes. In some exemplary embodiments, physician 30 may check whether the parameter has reached the target threshold by a visual comparison, for example, by comparing between bars 62 and 63.

In case the parameter has not yet reached the target threshold, the method loops back to step 104 for applying a second IRE pulse followed by acquiring a third bipolar signal having a third amplitude, and estimating, in steps 106 and 108, whether or not the third amplitude has reached the target threshold.

At a procedure concluding step 110, in case the parameter has reached the target threshold, e.g., after applying any suitable number of IRE ablation pulses, physician 30 concludes the IRE ablation procedure and retracts catheter 21 from patient heart 26.

In some cases, after performing the loop of steps 104, 106 and 108 several times, the parameter indicative of the progress of the ablation procedure may not reach the target threshold. Therefore, in other exemplary embodiments, processor 41 may suggest physician 30 to apply to the target tissue a set of one or more complementary radiofrequency (RF) ablation pulses, using catheter 21, or any other suitable catheter, which is configured to carry out RF ablation procedures.

Although the exemplary embodiments described herein mainly address improvement of irreversible electroporation (IRE) ablation procedures, the methods and systems described herein can also be used in other applications, such as in any other ablation procedures, such as but not limited to RF ablation, applied to any suitable organ of patient 28.

It will thus be appreciated that the exemplary embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECTS OF THE INVENTION

1. A method for estimating the progress of irreversible electroporation ablation, the method comprising:
   coupling, to a target tissue, at least a pair of electrodes of an ablation catheter;
   receiving from the pair of electrodes, at a first stage of an ablation procedure, a first bipolar signal having a first amplitude;
   receiving from the pair of electrodes, at a second stage of the ablation procedure, a second bipolar signal having a second amplitude; and
   estimating, based on the first and second amplitudes, at least a parameter indicative of a progress of the ablation procedure.
2. The method according to aspect 1, wherein estimating the parameter comprises displaying the first and second amplitudes on a same graph.
3. The method according to aspect 2, wherein displaying the first and second amplitudes comprises displaying, on the same graph, a first bar indicative of the first amplitude and a second bar indicative of the second amplitude.
4. The method according to aspect 3, and comprising receiving from an additional pair of electrodes of the ablation catheter, an additional first bipolar signal having an additional first amplitude and an additional second bipolar signal having an additional second amplitude, and comprising, displaying on the same graph an additional first bar indicative of the additional first amplitude, and an additional second bar indicative of the additional second amplitude.
5. The method according to aspect 4, wherein the pair of electrodes is coupled to the target tissue at a first site, and the additional pair of electrodes is coupled to the target tissue at a second site, different from the first site, and comprising, based on the same graph, applying, to the target tissue: (i) a first irreversible electroporation (IRE) pulse at the first site, and (ii) a second IRE pulse at the second site.
6. The method according to aspect 5, wherein applying the first and second IRE pulses comprises applying the first IRE pulse that differs from the second IRE pulse.
7. The method according to aspect 3, wherein displaying the first and second amplitudes comprises displaying the first and second bars overlaying one another.
8. The method according to aspect 1, wherein receiving at the first stage comprises receiving the first bipolar signal before applying an irreversible electroporation (IRE) pulse to the target tissue, and receiving at the second stage comprises receiving the second bipolar signal after applying the IRE pulse to the target tissue.
9. The method according to aspect 8, and comprising, based on the estimated parameter, checking whether to apply to the target tissue, an additional IRE pulse, and wherein, in case the additional IRE pulse is required, the method comprising, at a third stage of the ablation procedure: (i) based on the estimated parameter, applying to the target tissue the additional IRE pulse, (ii) receiving, from the pair of electrodes, a third bipolar signal having a third amplitude, and (iii) estimating at least the parameter based on the first and third amplitudes.
10. The method according to aspect 1, wherein estimating the parameter comprises displaying at least one of: (i) a first numerical difference between the first and second amplitudes, and (ii) a second numerical difference between the second amplitude and a target threshold.

## Claims

1. A system for estimating the progress of irreversible electroporation ablation, the system comprising:
a processor, which is configured to receive, from at least a pair of electrodes of an ablation catheter that are coupled to a target tissue: (a) at a first stage of an ablation procedure, a first bipolar signal having a first amplitude, and (b) at a second stage of the ablation procedure, a second bipolar signal having a second amplitude, and wherein, based on the first and second amplitudes, the processor is configured to estimate at least a parameter indicative of a progress of the ablation procedure; and
a display, which is configured to display the parameter.

2. The system according to claim 1, wherein the processor is configured to display the first and second amplitudes on a same graph for estimating the parameter.

3. The system according to claim 2, wherein the processor is configured to display on the same graph, a first bar indicative of the first amplitude and a second bar indicative of the second amplitude.

4. The system according to claim 3, wherein the processor is configured to receive from an additional pair of electrodes of the ablation catheter, an additional first bipolar signal having an additional first amplitude and an additional second bipolar signal having an additional second amplitude, and to display on the same graph an additional first bar indicative of the additional first amplitude, and an additional second bar indicative of the additional second amplitude.

5. The system according to claim 4, wherein the pair of electrodes is coupled to the target tissue at a first site, and the additional pair of electrodes is coupled to the target tissue at a second site, different from the first site, and wherein, based on the same graph, the processor is configured to control a pulse generator to apply to the target tissue: (i) a first irreversible electroporation (IRE) pulse at the first site, and (ii) a second IRE pulse at the second site.

6. The system according to claim 5, wherein the processor is configured to control the pulse generator to apply the first and second IRE pulses different from one another.

7. The system according to claim 3, wherein the processor is configured to display the first and second bars overlaying one another.

8. The system according to claim 1, wherein the processor is configured to receive the first bipolar signal before applying an irreversible electroporation (IRE) pulse to the target tissue, and to receive the second bipolar signal after applying the IRE pulse to the target tissue.

9. The system according to claim 8, wherein, based on the estimated parameter, the processor is configured to check whether to apply to the target tissue, an additional IRE pulse, and wherein, in case the additional IRE pulse is required, at a third stage of the ablation procedure the processor is configured: (i) based on the estimated parameter, to apply the additional IRE pulse to the target tissue, (ii) to receive, from the pair of electrodes, a third bipolar signal having a third amplitude, and (iii) to estimate at least the parameter based on the first and third amplitudes.

10. The system according to claim 1, wherein the processor is configured to estimate at least the parameter by displaying on the display, at least one of: (i) a first numerical difference between the first and second amplitudes, and (ii) a second numerical difference between the second amplitude and a target threshold.
